# EUROPÄISCHE PATENTANMELDUNG

(11) **EP 3 878 486 A1**
(43) Veröffentlichungstag der Anmeldung: **15.09.2021**
(21) Anmeldenummer: 20163026.6
(22) Anmeldetag: 13.03.2020
(51) Int. Cl.: A61M 1/00

(54) **VORRICHTUNG ZUR UNTERDRUCKBEHANDLUNG UND INSTILLATION VON WUNDEN**

(71) Anmelder: Medela Holding AG, 6340 Baar (CH)
(72) Erfinder: Bannwart, Lukas, 6343 Rotkreuz (CH)
(74) Vertreter: Grünecker Patent- und Rechtsanwälte PartG mbB

(57) **Zusammenfassung**

Die Erfindung betrifft eine Vorrichtung zur Unterdruckbehandlung und Instillation von Wunden bereit. Die Vorrichtung umfasst einen Verband zur fluiddichten Abdeckung einer Wunde, eine Abflussleitung zum Absaugen von Fluid von der Wunde, ein Unterdruckaggregat zum Erzeugen eines Unterdrucks in der Abflussleitung, eine Instillationsleitung zum Bereitstellen eines Instillationsfluids, eine optionale Spülleitung zum Bereitstellen eines Spülfluids und eine mit dem Verband verbundene Hauptleitung. Die Hauptleitung (60) weist ein einzelnes Lumen auf und ist über ein Anschlusselement (64) mit zwei der folgenden Leitungen, Abflussleitung (20), Instillationsleitung (40) und Spülleitung (50), verbindbar. Die Erfindung betrifft außerdem ein Verfahren zum Betrieb dieser Vorrichtung.

## Beschreibung

### TECHNISCHES GEBIET

Die vorliegende Erfindung betrifft eine Vorrichtung zur Unterdruckbehandlung und Instillation von Wunden. Außerdem betrifft die Erfindung ein Verfahren zum Betrieb einer solchen Vorrichtung.

### STAND DER TECHNIK

Im medizinischen Bereich ist es seit langem bekannt, dass die Wundheilung insbesondere bei großen und/oder schwer heilenden Wunden mittels Unterdruckbehandlung des Wundbetts verbessert werden kann (sog. NPWT - Negative-Pressure Wound Therapy). Dabei werden Körperfluide oder Sekrete, die auch als Exsudat bezeichnet werden, aus dem Wundbett mittels einer Pumpe abgesaugt. Während der Unterdruckbehandlung kann die Wundheilung zusätzlich durch Zuführen eines Instillationsfluids zum Wundbett positiv beeinflusst werden. Derartige Methoden sind als Unterdruck-Wundtherapie mit kombinierter Instillation oder Irrigation bekannt. Je nach Typ und Größe der Wunde erfolgen die Absaugung und die Zuführung dabei gleichzeitig, nacheinander und/oder abwechslungsweise intermittierend.

Bei dem zuzuführenden Instillationsfluid kann es sich beispielsweise um eine physiologische oder nicht physiologische Kochsalzlösung, ein Arzneimittel oder um ein Gemisch davon handeln. Das Instillationsfluid kann zum Beispiel zur Förderung der Wundheilung, zur Verhinderung von Infektionen, zur lokalen Anästhesie oder zur Verbesserung der Blutgerinnung durch Hemmung der Fibrinolyse dienen. Das Zuführen des Instillationsfluids kann somit zur Spülung oder therapeutischen, diagnostischen und/oder präventiven Zwecken dienen.

Generelle Beschreibungen der kombinierten Unterdruck- und Instillationsbehandlung von Wunden finden sich zum Beispiel in der Veröffentlichung Bobkiewicz, Adam et al.; Negative pressure wound therapy with Instillation (NPWTi): Current Status, recommendations and perspectives in the context of modern wound therapy; Negative Pressure Wound Therapy Journal, [S.I.], v. 3, n. 1, apr. 2016; oder in der Veröffentlichung Gupta, Subhas et al.; Clinical recommendations and practical guide for negative pressure wound therapy with Instillation; Int Wound J, 2016 Apr; 13(2): 159-174.

Oft wird zum Zuführen des Instillationsfluids ähnlich wie bei der herkömmlichen Infusion ein mit dem zuzuführenden Fluid gefüllter Flüssigkeitsbeutel oder eine Flasche erhöht über der zu behandelnden Körperstelle angeordnet, so dass das Fluid aufgrund des hydrostatischen Druckes durch eine Zuführleitung der zu behandelnden Stelle zugeführt wird. Separat dazu werden die Körperfluide von einer Vakuumpumpe via eine entsprechende Leitung abgesaugt.

Um eine bessere Einstellung und Regelung beim Zuführen des Instillationsfluids zu ermöglichen, und/oder um unabhängig von der Anordnung und insbesondere der Höhenlage des mit dem Fluid gefüllten Flüssigkeitsbehälters zu sein, sind auch Systeme hinlänglich bekannt, bei denen die Zuführung des Fluids zum Körper mittels einer Pumpe, insbesondere einer sog. Peristaltik- oder Schlauchpumpe erfolgt.

Zur Überwachung der Therapie wird in vielen Systemen des Standes der Technik laufend oder in regelmäßigen Zeitabständen der im Wundbereich herrschende Druck gemessen. Dies wird beispielsweise mittels einer Hilfsleitung erreicht, welche im Bereich des patientenseitigen Endes der Sekretleitung seitlich in diese mündet, oder welche separat zur Sekretleitung durch die Wundabdeckung geführt ist und direkt zum Wundbereich hin mündet.

Diese Hilfsleitung kann zusätzlich die Funktion einer Spülleitung übernehmen, mit der ein Spülfluid zur Wunde und durch die Sekretleitung geleitet wird, um diese beispielsweise von Rückständen des Instillationsfluids oder des Exsudats zu reinigen.

Die WO 2018/130466 A1 offenbart beispielsweise eine Vorrichtung zum Heilen von Wundgewebe mittels Unterdruckbehandlung, bei welcher ein Instillationsfluid mittels einer Instillationsleitung, die direkt in das Wundbett mündet, einer Wunde zugeführt wird und bei welcher die Absaugung von Fluiden von dem Wundbett mittels einer separaten Sekretleitung erfolgt. Zusätzlich zu der Instillations- und der Sekretleitung kann eine Hilfsleitung vorgesehen sein, die entweder in Nähe des Wundbetts in die Sekretleitung oder direkt in das Wundbett mündet. Die Hilfsleitung erlaubt es, den Druck in der Sekretleitung bzw. an der Wunde zu messen oder die Sekretleitung zu spülen.

Die EP 3 235 526 A1 offenbart eine Vorrichtung zur Unterdruckbehandlung und Instillation von Wunden, bei der ein Unterdruckaggregat über eine mehrlumige Verbindung an einen Wundverband angeschlossen ist. Über ein Lumen der Verbindung wird der Druck im Wundverband gemessen. Ein primäres Lumen dient zum Transport des abgesaugten Fluids. Ebenso ist ein Instillationsbehälter über mehrere Fluidlumen an den Wundverband angeschlossen, wobei ein Lumen zum Fluidtransport und ein weiteres zur Druckmessung dient.

Eine Vorrichtung mit einer Sekretleitung zum Absaugen von Wundsekret, einer Instillationsleitung zum Zuführen von Instillationsfluid und einer Hilfsleitung zum Spülen der Sekretleitung und zur Druckmessung ist in US 2019/0275219 A1 offenbart.

Weitere Vorrichtungen, bei denen eine zusätzliche Hilfsleitung zur Druckmessung an den Wundverband angeschlossen sind, sind in EP 2 714 120 B1, US 2011/0178479 A1 und EP 0 865 304 B1 offenbart.

### ARSTELLUNG DER ERFINDUNG

Es ist eine Aufgabe der vorliegenden Erfindung, eine Vorrichtung zur Unterdruckbehandlung und Instillation von Wunden bereitzustellen, mit der ein einfacherer Anschluss des Wundverbands an die Vorrichtung und damit ein einfacherer Wechsel des Wundverbands möglich ist. Zusätzlich soll die Reinigung der Vorrichtung auch im Betrieb erleichtert werden und somit höheren hygienischen Anforderungen genügen. Des Weiteren sollen Störungen im Betriebsablauf, insbesondere eine Blockade der Fluidverbindung zwischen Verband und Vorrichtung erkannt werden können.

Zur Lösung dieser Aufgabe stellt die Erfindung eine Vorrichtung zur Unterdruckbehandlung und Instillation von Wunden bereit. Die Vorrichtung umfasst einen Verband zur fluiddichten Abdeckung einer Wunde, eine Abflussleitung zum Absaugen von Fluid von der Wunde, ein Unterdruckaggregat zum Erzeugen eines Unterdrucks in der Abflussleitung, eine Instillationsleitung zum Bereitstellen eines Instillationsfluids, eine optionale Spülleitung zum Bereitstellen eines Spülfluids und eine mit dem Verband verbundene Hauptleitung. Die Hauptleitung weist ein einzelnes Lumen auf und ist über ein Anschlusselement mit zwei der folgenden Leitungen, Abflussleitung, Instillationsleitung und Spülleitung, verbindbar.

Die Erfindung ist dadurch gekennzeichnet, dass zwei der folgenden Leitungen, Abflussleitung, Instillationsleitung und Spülleitung, zu einer einlumigen Hauptleitung zusammengefasst und mittels der Hauptleitung an den Verband angeschlossen werden können. Die zwei Leitungen sind dabei vorzugsweise über das Anschlusselement mit der Hauptleitung verbunden. Beispielsweise werden die Abfluss- und die Instillationsleitung, oder die Instillations- und die Spülleitung über das Anschlusselement zu einer einlumigen Hauptleitung zusammengefasst und mittels der Hauptleitung an den Verband angeschlossen. Die jeweils dritte Leitung ist separat an den Verband angeschlossen, so dass der Verband insgesamt über nicht mehr als zwei Lumina mit der Vorrichtung verbindbar ist.

Auf die Spülleitung kann verzichtet werden. In diesem Fall werden die Abfluss- und die Instillationsleitung über das Anschlusselement zu der einlumigen Hauptleitung zusammengefasst, so dass der Verband lediglich über ein direkt angeschlossenes Lumen mit der Vorrichtung verbunden werden muss.

Umfasst die Vorrichtung jeweils eine Abfluss-, Instillations- und Spülleitung, werden zwei dieser Leitungen über das Anschlusselement zu der einlumigen Hauptleitung zusammengefasst, während die dritte Leitung direkt mit dem Verband verbunden wird. Auf diese Weise ist der Verband lediglich über zwei direkt angeschlossene Lumina mit der Vorrichtung verbunden.

Bei herkömmlichen Vorrichtungen zur Unterdruckbehandlung und Instillation von Wunden sind hingegen vollständig voneinander getrennte Abfluss-, Instillations- und Spülleitungen vorgesehen, die separat an den Wundverband angeschlossen werden. Damit müssen insgesamt drei Fluidleitungen an den Wundverband angeschlossen werden.

Die erfindungsgemäße Vorrichtung erfordert hingegen lediglich den Anschluss von nicht mehr als zwei Fluidleitungen an den Wundverband. Auf diese Weise kann für die kombinierte Unterdruck- und Instillationsbehandlung die gleiche Art von Wundverband verwendet werden wie für die reine Unterdruck- oder Instillationstherapie, bei der nur zwei Fluidleitungen an den Wundverband angeschlossen werden. Mit der erfindungsgemäßen Vorrichtung wird außerdem der Wechsel des Wundverbands erleichtert, weil lediglich die Verbindung zu zwei Fluidleitungen getrennt werden muss. Dadurch, dass die Hauptleitung mit dem Spülfluid gereinigt und von Resten des Instillationsfluids oder des Exsudats befreit werden kann, ergeben sich des Weiteren hygienische Vorteile. Des Weiteren kann durch eine Messung des Drucks in der Spülleitung im laufenden Betrieb eine Blockade im fluidführenden System festgestellt werden.

Weitere Vorteile der erfindungsgemäßen Vorrichtungen sowie bevorzugte Ausführungsformen werden im Folgenden beschrieben. Mit einer "Leitung", beispielsweise einer Abfluss- oder Hauptleitung, ist im Rahmen der vorliegenden Erfindung eine Fluidverbindung mit einem einzelnen Lumen gemeint. Eine Verbindung mit mehreren Lumina, beispielsweise eine zweilumige Schlauchverbindung, umfasst im Rahmen dieser Erfindung mehrere Leitungen.

Eine Verbindung zwischen zwei Leitungen bzw. zwischen einer Leitung und einem Anschlusselement meint im Rahmen der vorliegenden Erfindung stets eine fluidische Verbindung, also eine Verbindung, die eine Weiterleitung von Fluid zwischen den verbundenen Bauteilen erlaubt.

Mittels der Instillationsleitung kann der Wunde ein Instillationsfluid zugeleitet werden. Das Instillationsfluid dient beispielsweise dazu, nekrotisches Gewebe von der Wunde zu lösen, die Wunde zu reinigen und die Wundflüssigkeit zu verdünnen. Damit trägt das Instillationsfluid zum Debridement der Wunde bei. Das Instillationsfluid kann aber auch zum Einbringen eines Medikaments oder zur lokalen Betäubung des Wundbereichs dienen. Dabei kann das Instillationsfluid zusätzlich zum Beispiel mit einer definierten Temperatur, UV-Strahlung und/oder einer bestimmten chemischen Zusammensetzung oder Mischung vorkonditioniert werden. Das Instillationsfluid kann gasförmig oder flüssig sein. Vorzugsweise handelt es sich bei dem Instillationsfluid um eine wässrige Lösung, beispielsweise eine physiologische Kochsalzlösung. Das Instillationsfluid kann auch einen oder mehrere Wirkstoffe enthalten, insbesondere medizinische Wirkstoffe zur Förderung der Wundheilung. Bei dem Instillationsfluid kann es sich auch um Luft, mit Sauerstoff angereicherte Luft oder Stickstoff handeln. Das Instillationsfluid kann auch ein Aerosol sein. Das Aerosol umfasst vorzugsweise ein Trägergas, wie Luft oder Stickstoff, und eine darin zerstäubte wässrige Lösung, wie z.B. eine Wirkstofflösung.

Die Instillationsleitung umfasst vorzugsweise ein Ventil (Rückflussventil), mit dem ein Rückfluss von Fluid, insbesondere von Instillations- und Spülfluid aus der Hauptleitung, in die Instillationsleitung verhindert werden kann. Die Funktion dieses Ventils kann in einer alternativen Ausführungsform auch von einer in der Instillationsleitung angeordneten Peristaltikpumpe übernommen werden, welche beispielsweise die Fluidleitung abklemmt und auf diese Weise einen Rückfluss von Fluid in die Instillationsleitung verhindert. Es können auch gleichzeitig eine Peristaltikpumpe und ein Rückflussventil vorgesehen sein. In bestimmten Fällen kann die Abdichtwirkung der Peristaltikpumpe nicht ausreichen, so dass es bei einem im Verband anliegenden Unterdruck zu einem ungewollten Durchtritt von Instillationsfluid in den Verband kommen kann. Um dies zu verhindern, kann ein zusätzliches, regelbares Ventil vorgesehen sein, dass die Fluidverbindung zwischen der Peristaltikpumpe und dem Verband in beiden Richtungen unterbricht. Diese Funktion kann auch durch das zuvor genannte Rückflussventil übernommen werden.

Die Instillationsleitung ist vorzugsweise an einen Instillationsbehälter angeschlossen. Dieser ist vorzugsweise austauschbar mit der Instillationsleitung verbunden. Der Instillationsbehälter dient zur Aufnahme eines Vorrats an Instillationsfluid. Der Instillationsbehälter kann beispielsweise aus starrem oder flexiblem Kunststoff bestehen. Beispielsweise handelt es sich bei dem Instillationsbehälter um einen Kunststoffbeutel oder eine Kunststoffflasche. Vorzugsweise besteht der Instillationsbehälter zumindest teilweise aus transparentem Material, insbesondere transparentem Kunststoffmaterial, damit der Füllstand von außen eingesehen werden kann.

In einer weiteren Ausführungsform kann der Instillationsbehälter relativ zum Verband so angeordnet werden, dass das Instillationsfluid mittels Schwerkraft zur Wunde geleitet wird. Zu diesem Zweck kann die Vorrichtung eine Halterung für den Instillationsbehälter umfassen, beispielsweise eine Aufhängung, mit welcher der Instillationsbehälter erhöht gegenüber dem Verbund befestigt werden kann. In diesem Fall kann die Instillationsleitung einen Tropfenzähler aufweisen, mit dem die Menge an zugeführtem Instillationsfluid bestimmt werden kann. Der Fluidfluss durch die Instillationsleitung kann bei dieser Ausführungsform durch eine zusätzlich vorgesehene, unten beschriebene Pumpe gesteuert werden.

Vorzugsweise umfasst die Vorrichtung eine Spülleitung zusätzlich zu der Abfluss- und der Instillationsleitung. Vorzugsweise sind in dieser Ausführungsform entweder die Abfluss- und die Instillationsleitung oder die Instillations- und die Spülleitung mit dem Anschlusselement verbindbar. Besonders bevorzugt sind diese Leitungen mit dem Anschlusselement verbunden. Am meisten bevorzugt ist die Ausführungsform, bei der die Instillations- und die Spülleitung mit dem Anschlusselement und damit mit der Hauptleitung verbunden sind und die Abflussleitung getrennt davon an den Verband angeschlossen ist.

Mittels der Spülleitung kann der Wunde ein Spülfluid zugeleitet werden. Das Spülfluid kann gasförmig oder flüssig sein. Vorzugsweise handelt sich bei dem Spülfluid um Luft. Die Spülleitung ermöglicht eine Druckangleichung und eine Durchspülung von Hauptleitung, Verband und Abflussleitung. Vorzugsweise steht die Spülleitung über ein regelbares Belüftungsventil in Fluidkommunikation mit der Umgebungsluft.

Die Spülleitung umfasst vorzugsweise ein Ventil, mit dem ein Rückfluss von Fluid, insbesondere Instillations- und Spülfluid aus der Hauptleitung, in die Spülleitung verhindert werden kann. Bei diesem Ventil kann es sich auch um eine Membran handeln, die bei Kontakt mit Flüssigkeit verschließt und den Fluidfluss unterbricht. Eine derartige Membran kann beispielsweise ein Material umfassen, dass bei Kontakt mit Feuchtigkeit aufquillt und somit eine Fluidundurchlässige Barriere bildet. Alternativ kann eine derartige Membran als hydrophobe PTFE-Membran ausgeführt sein, die nur Luft passieren lässt. Eine solche Ausführung der Membran kann auch als Filter zur Vermeidung von mikrobiologischer Kontamination der Spülleitung wirken.

In einer bevorzugten Ausführungsform ist in der Spülleitung ein Drucksensor angeordnet. Mit Hilfe dieses Sensors kann der in der Spülleitung anliegende Unterdruck und damit auch der im Wundverband anliegende Unterdruck kontinuierlich überwacht werden.

Die Spülleitung umfasst außerdem vorzugsweise mindestens eine Filtereinheit, um mikrobiologische Kontaminationen aus dem Spülfluid zu entfernen. In dem Fall, dass die Spülleitung über ein regelbares Belüftungsventil in Fluidkommunikation mit der Umgebungsluft steht, wird die Umgebungsluft vorzugsweise über eine Filtereinheit geleitet, um mikrobiologische Kontaminationen aus der Umgebungsluft zu entfernen. Diese Filtereinheit ist vorzugsweise zwischen dem Belüftungsventil und dem mit der Umgebung in Kontakt stehenden Ende der Spülleitung angeordnet. In dem Fall, dass die Spülleitung über einen Drucksensor verfügt, ist vorzugsweise eine zusätzliche Filtereinheit zwischen dem Drucksensor und dem der Hauptleitung zugewandten Ende der Spülleitung angeordnet.

In einer bevorzugten Ausführungsform umfasst die Vorrichtung eine Pumpe zur Erzeugung eines Fluidflusses in der Hauptleitung. Dabei handelt es sich vorzugsweise um eine Peristaltikpumpe. Mit Hilfe der Pumpe wird ein Fluidfluss in der Hauptleitung erzeugt, um das Instillations- und/oder das Spülfluid in Richtung der Wunde zu leiten. Dies ermöglicht eine bessere Regelung beim Zuführen von Spül- und/oder Instillationsfluid während der Behandlung. Außerdem ist die Flussrate unabhängig von der relativen Anordnung von Instillationsbehälter und Verband einstellbar. Somit ist die Pumpe vorzugsweise auch dann vorgesehen, wenn der Instillationsbehälter oberhalb des Verbandes angeordnet ist, um einen hydrostatischen Druck im Instillationsfluid zu erzeugen. Die Pumpe kann in der Hauptleitung, der Spülleitung, der Instillationsleitung oder dem Anschlusselement, welches Zufluss-, Instillations- und Spülleitung miteinander verbindet, angeordnet sein. In einer bevorzugten Ausführungsform ist die Pumpe in der Instillationsleitung angeordnet und dient somit zum Erzeugen eines Fluidflusses in dem Instillationsfluid. Eine Pumpe kann auch dem Fall vorgesehen sein, dass das Instillationsfluid mittels Schwerkraft zur Wunde geleitet wird, um den Fluidfluss zusätzlich zu steuern.

Die Pumpe wird insbesondere dann eingesetzt, wenn es sich bei dem Instillations- und/oder dem Spülfluid um eine Flüssigkeit handelt. Bei einem gasförmigen Fluid kann auf eine Pumpe verzichtet werden, da ein Fluidfluss in dem gasförmigen Fluid mittels des im Verband anliegenden Unterdrucks erzeugt werden kann. So wird in einer bevorzugten Ausführungsform die Pumpe zum Erzeugen eines Fluidflusses in dem vorzugsweise flüssigen Instillationsfluid eingesetzt, während der Fluidfluss in dem vorzugsweise gasförmigen Spülfluid durch Unterdruck erzeugt wird.

Geeignete Peristaltikpumpen sind beispielsweise in EP 3 603 689 A1, US 2019/0275218 A1 und WO 2018/172217 A1 beschrieben.

Die Pumpe ist vorzugsweise mit einer Steuereinheit verbunden, mit der die Pumpleistung zeitlich angepasst werden kann. Zusätzlich kann die Pumpe mit einer Messeinrichtung verbunden sein, die auf Basis der Pumpleistung die zugeführte Menge an Instillations- und/oder Spülfluid bestimmt.

In einer bevorzugten Ausführungsform umfasst die Vorrichtung eine Einheit zur Steuerung des Fluidflusses durch die Instillationsleitung, mit der die Flussrate zeitlich gesteuert werden kann. Beispielsweise kann die Vorrichtung ein steuerbares Ventil in der Instillationsleitung umfassen, mit dem die Fluidverbindung zum Installationsbehälter unterbrochen werden kann. Es ist auch möglich, die oben genannte Pumpe zur Erzeugung eines Fluidflusses in der Hauptleitung an eine Steuereinheit zur Einstellung der Pumpleistung zu koppeln.

In einer bevorzugten Ausführungsform umfasst die Vorrichtung eine Einheit zur Steuerung des Fluidflusses durch die Hauptleitung, so dass wechselweise die Fluidverbindung zu einer der angeschlossenen Leitungen unterbrochen werden kann. Beispielsweise kann entweder das Instillationsfluid oder das Spülfluid durch die Hauptleitung geleitet werden. Bei der Einheit zur Steuerung des Fluidflusses kann es sich beispielsweise um zwei gleichzeitig steuerbare Ventile handeln, die jeweils den Fluidfluss durch die Instillationsleitung bzw. die Spülleitung freigeben bzw. blockieren. Ebenso kann das Anschlusselement mit einer Vorrichtung ausgestattet sein, die wechselweise die Fluidverbindung von der Hauptleitung zur Instillationsleitung oder zur Spülleitung freigibt, beispielsweise einem Drehventil.

Eine entsprechende Vorrichtung kann vorgesehen sein, wenn das Anschlusselement mit der Abfluss- und der Instillationsleitung verbunden ist. Die Abflussleitung dient zum Absaugen von Fluiden von der Wunde bzw. aus dem Wundraum. Das abgesaugte Fluid kann gasförmig oder flüssig sein. Insbesondere handelt es sich bei dem abgesaugten Fluid um als Exsudat bezeichnete Körperflüssigkeit, die während des Verlaufs der Unterdruckbehandlung von der Wunde abgesondert wird. Die Abflussleitung dient allerdings ebenso zum Absaugen des Instillations- und des Spülfluids. Die zum Absaugen erforderliche Fluidströmung wird durch den in der Abflussleitung anliegenden Unterdruck erzeugt.

Vorzugsweise ist in der Abflussleitung ein Abflussventil angeordnet. Das Abflussventil kann steuerbar sein, um die Fluidverbindung innerhalb der Abflussleitung zu öffnen oder zu unterbrechen. Dieses Ventil erlaubt beispielsweise, einen gegebenenfalls an die Abflussleitung angeschlossenen Sammelbehälterzu belüften, während im Verband noch ein Unterdruck anliegt.

Vorzugsweise ist das Abflussventil ein Einwegventil, das die Fluidkommunikation in der Richtung vom Verband weg zulässt und die Fluidkommunikation in Richtung zum Verband hin blockiert.

In einer bevorzugten Ausführungsform umfasst die Vorrichtung sowohl Abfluss- und Instillationsleitung als auch eine Spülleitung, wobei zwei dieser Leitungen über das Anschlusselement mit der Hauptleitung verbunden werden. Vorzugsweise bilden die Hauptleitung und die jeweilige Leitung, die nicht mit dem Anschlusselement verbunden ist, zusammen zumindest abschnittweise eine zweilumige Fluidleitung. Beispielsweise sind Instillationsleitung und Spülleitung mit der Hauptleitung verbunden und die Abflussleitung sowie die Hauptleitung bilden zusammen zumindest abschnittsweise eine zweilumige Fluidleitung. Dies erleichtert die Handhabung der Vorrichtung, insbesondere den Anschluss des Verbands an die Haupt- und Abschlussleitung, da die zweilumige Leitung effektiv wie eine einzelne Schlauchverbindung gehandhabt werden kann. So kann beispielsweise die zweilumige Leitung in einem einzigen Arbeitsschritt mittels eines geeigneten Anschlusselements mit dem Verband verbunden werden. Außerdem kann eine Kopplung in der zweilumigen Leitung vorgesehen sein, mittels derer diese von der Vorrichtung getrennt werden kann.

In einer bevorzugten Ausführungsform ist die Abflussleitung mit einem Sammelbehälter zur Aufnahme des von der Wunde abgesaugten Fluids verbunden. Der Sammelbehälter besteht vorzugsweise aus Kunststoff, besonders bevorzugt aus einem starren Kunststoff. Vorzugsweise besteht der Sammelbehälter zumindest teilweise aus transparentem Material, so dass der Füllstand von außen eingesehen werden kann. Der Sammelbehälter weist vorzugsweise einen Anschluss zur Verbindung mit der Abflussleitung und einen weiteren Anschluss zur Verbindung mit dem Unterdruckaggregat auf. Der Anschluss zur Verbindung mit der Abflussleitung ist vorzugsweise mit einem Abflussventil gesichert, mit dem ein Rückfluss des abgesaugten Fluids aus dem Sammelbehälter in die Abflussleitung verhindert werden kann. Das Abflussventil kann steuerbar sein, um die Fluidverbindung zwischen Verband und Sammelbehälter zu öffnen oder zu unterbrechen. Vorzugsweise ist das Abflussventil ein Einwegventil, das die Fluidkommunikation in Richtung des Sammelbehälters zulässt und die Fluidkommunikation in Richtung des Verbands blockiert. Der Anschluss zur Verbindung mit dem Unterdruckaggregat weist vorzugsweise eine Filtereinheit auf, um eine wechselseitige Kontamination von Sammelbehälter und der Verbindung zum Unterdruckaggregat zu verhindern.

Vorzugsweise weist die Vorrichtung einen Drucksensor auf, um den in der Abflussleitung und/oder dem Sammelbehälter anliegenden Unterdruck zu messen. Dieser Drucksensor kann in der Abflussleitung, dem Sammelbehälter oder der Verbindung zwischen Sammelbehälter und Unterdruckaggregat angeordnet sein. Besonders bevorzugt ist der Drucksensor in der Verbindung zwischen Sammelbehälter und Unterdruckaggregat angeordnet.

In einer Ausführungsform weist die Vorrichtung eine Bypassleitung auf, welche eine Fluidkommunikation zwischen der Abflussleitung und der Spülleitung herstellt. Vorzugsweise ist in der Bypassleitung ein Bypassventil angeordnet, welches die Fluidverbindung zwischen Abflussleitung und Spülleitung blockiert bzw. öffnet. Die Bypassleitung kann beispielsweise an einer Position zwischen Verband und dem oben beschriebenen Sammelbehälter in die Abflussleitung münden. Alternativ kann die Bypassleitung in die Verbindungsleitung zwischen Sammelbehälter und Unterdruckaggregat münden. In dem Fall, dass die Abflussleitung ein oben beschriebenes Abflussventil umfasst, mündet die Bypassleitung vorzugsweise auf der dem Verband abgewandten Seite des Abflussventils in die Abflussleitung. Hinsichtlich der Spülleitung und in dem Fall, dass diese ein oben beschriebenes Belüftungsventil umfasst, mündet die Bypassleitung auf der dem Verband abgewandten Seite des Belüftungsventils in die Spülleitung.

Die Bypassleitung ermöglicht ein Belüften der Abflussleitung und des optional daran angeschlossenen Sammelbehälters zu Beginn der Unterdrucktherapie. Sofern die Abflussleitung mit einem Abflussventil und die Spülleitung mit einem Belüftungsventil gesichert sind, kann die Belüftung erfolgen, während am Verband bereits ein Unterdruck anliegt.

Bei dem Verband handelt es sich um einen für die Unterdruckbehandlung von Wunden geeigneten Verband, welcher eine Wunde unter Bildung eines Wundraums fluiddicht, insbesondere gasdicht, abschließen kann und eine Fluidkommunikation zwischen dem Wundraum und einer Unterdruckquelle und mindestens einer Fluidquelle ermöglicht. Der Verband umfasst vorzugsweise eine selbstklebende Abdeckfolie, mit der eine Wunde unter Bildung des Wundraums fluiddicht abgedeckt werden kann. Der Verband kann außerdem eine innerhalb des Wundraums angeordnete Wundauflage umfassen, beispielsweise eine textile oder aus Schaumstoff bestehende Wundauflage. Besonders bevorzugt ist eine Wundauflage aus offenzelligem Polymerschaumstoff. Die Wundauflage kann ein- oder mehrlagig ausgebildet sein. Vorzugsweise weist die Wundauflage eine antimikrobiell ausgerüstete Wundkontaktschicht auf, beispielsweise eine Schicht aus silberhaltigem Polyamidgewebe.

Der Anschluss von Zufluss- und Abflussleitung an den Verband erfolgt beispielsweise dadurch, dass die jeweiligen Enden der Leitungen unter der Abdeckfolie des Verbandes zum Wundraum hindurch geschoben werden. Alternativ kann der Verband einen oder mehrere geeignete Anschlüsse aufweisen, die mit den jeweiligen Enden der Leitungen gekoppelt werden. Beispielsweise können die Leitungen auf geeignete Anschlusselemente aufgesteckt werden. In einer bevorzugten Ausführungsform weist der Verband einen Anschluss mit zwei Kanälen auf, über die jeweils die Zufluss- und die Abflussleitung angeschlossen werden. In einer alternativen Ausführungsform weist der Verband zwei getrennte Anschlüsse mit jeweils einem Kanal zur Verbindung mit der Zufluss- bzw. der Abflussleitung auf.

Das Unterdruckaggregat zum Erzeugen des Unterdrucks ist vorzugsweise eine Vakuumpumpe. Mit dem Unterdruckaggregat wird ein Unterdruck in der Abflussleitung erzeugt, der sich in den von dem fluiddichten Verband gebildeten Wundraum und die daran angeschlossene Hauptleitung fortsetzt. Der somit im Wundraum anliegende Unterdruck fördert die Wundheilung und erlaubt außerdem ein Absaugen von Fluid aus dem Wundraum. Bei dem Unterdruckaggregat handelt es sich vorzugsweise um eine Membranpumpe. Eine Membranpumpe ist besonders geeignet, da sie einen oszillierenden Druckverlauf erzeugt, der sich positiv auf die Wundheilung auswirkt. Alternativ dazu können auch Vorkehrungen vorgesehen sein, ein Oszillieren des Unterdrucks zu vermeiden, so dass der im Wundraum anliegende Unterdruck zeitlich konstant ist. Die Vorrichtung weist vorzugsweise eine Steuereinheit auf, mit der die Saugleistung des Unterdruckaggregats zeitlich gesteuert werden kann. Hierdurch wird beispielsweise ein intermittierendes Absaugen von Fluid aus dem Wundraum ermöglicht. Es hat sich gezeigt, dass ein intermittierendes Absaugen positiv auf die Wundheilung auswirken kann.

Die zuvor genannten Steuereinheiten, insbesondere die Steuereinheiten zur Anpassung der Pumpleistung und der Saugleistung sowie die Einheit zur Steuerung des Fluidflusses durch die Hauptleitung, sind vorzugsweise programmierbar, so dass der Betrieb der Vorrichtung nach einem vorgegebenen zeitlichen Ablauf gesteuert wird. Die genannten Steuereinheiten können zusätzlich einen oder mehrere Betriebsparameter der Vorrichtung überwachen und Betrieb der Vorrichtung, insbesondere die Pumpleistung, die Saugleistung und den Fluidfluss durch die Hauptleitung, in Abhängigkeit dieser Betriebsparameter steuern. Beispielsweise werden hierbei der Unterdruck im Verband und/oder einer der angeschlossenen Leitungen, oder der Füllstand eines der an die Vorrichtung angeschlossenen Fluidbehälter überwacht. Die genannten Steuereinheiten können zu diesem Zweck auch mit einer zentralen programmierbaren Steuereinheit verbunden, bzw. zu einer solchen zentralen Einheit zusammengefasst sein.

In einer bevorzugten Ausführungsform umfasst die Vorrichtung ein Gehäuse, welches das Unterdruckaggregat in sich aufnimmt. Das Gehäuse kann zudem eine Stromversorgung in sich aufnehmen, insbesondere ein Netzteil mit einem Anschluss an eine externe Stromquelle oder eine wiederaufladbare Batterie. Alternativ kann ein solches Netzteil außerhalb des Gehäuses angeordnet sein. In einer bevorzugten Ausführungsform umfasst die Vorrichtung eine wiederaufladbare Batterie, insbesondere einen Lithium-Ionen-Akkumulator, die innerhalb des Gehäuses angeordnet ist und über einen an der Außenseite des Gehäuses zugänglichen Stromanschluss geladen werden kann. Dies hat den Vorteil, dass die Vorrichtung tragbar und unabhängig von einem fest installierten Stromnetz einsetzbar ist.

In einer bevorzugten Ausführungsform verläuft die Spülleitung zumindest teilweise innerhalb des Gehäuses, wobei ein mit der Spülleitung verbundener, optionaler Drucksensor ebenfalls innerhalb des Gehäuses angeordnet sein kann. Zusätzlich umfasst das Gehäuse vorzugsweise eine an der Außenseite angeordnete Belüftungsvorrichtung, mit der die Spülleitung in Fluidkommunikation mit der Umgebungsluft gebracht wird.

In einer bevorzugten Ausführungsform verläuft die Instillationsleitung außerhalb des Gehäuses. Falls die Instillationsleitung mit dem Anschlusselement verbunden ist, kann an der Außenseite des Gehäuses ein Anschluss zur Verbindung der Instillationsleitung mit dem Anschlusselement vorgesehen sein. Vorzugsweise verbindet die Instillationsleitung den Instillationsbehälter mit dem Gehäuse.

Der Instillationsbehälter kann auch lösbar mit dem Gehäuse verbunden werden, so dass er im angeschlossenen Zustand eine Einheit mit dem Gehäuse bildet. Beispielsweise kann der Instillationsbehälter zusammen mit dem Sammelbehälter zur Aufnahme des von der Wunde abgesaugten Fluids eine Einheit bilden, wobei diese Einheit lösbar mit dem Gehäuse verbunden ist. In einer weiteren Ausführungsform ist der Instillationsbehälter innerhalb des Gehäuses angeordnet.

In einer bevorzugten Ausführungsform ist das Anschlusselement zur Verbindung von Instillations- und Spülleitung innerhalb des Gehäuses angeordnet. In diesem Fall kann das Gehäuse an der Außenseite einen Anschluss zur Verbindung des Anschlusselements mit der Hauptleitung aufweisen.

Ein optional vorhandener Sammelbehälter zur Aufnahme des von der Wunde abgesaugten Fluids ist vorzugsweise lösbar mit dem Gehäuse verbunden. Vorzugsweise bildet der Sammelbehälter im angeschlossenen Zustand eine Einheit mit dem Gehäuse. Vorzugsweise ist der Sammelbehälter über eine Schnappverbindung am Gehäuse befestigt.

Die Erfindung betrifft außerdem ein Verfahren zum Betrieb der oben beschriebenen Vorrichtung zur Unterdruckbehandlung und Instillation von Wunden. Das Verfahren umfasst die folgenden Schritte:
a) Befüllen des Verbands mit Instillationsfluid aus der Hauptleitung,
b) Hindurchleiten eines Spülfluids durch die Spülleitung, die Hauptleitung, den Verband und die Abflussleitung bei gleichzeitigem stetigen oder intermittierenden Betrieb des Unterdruckaggregats.

Schritt b) wird durch die erfindungsmäße Vorrichtung ermöglicht, bei der Spülleitung und Instillationsleitung gemeinsam in die Hauptleitung münden. Dadurch ist es möglich, in Schritt b) nicht nur den Verband und die Abflussleitung, sondern gleichzeitig auch die Instillationsleitung zu spülen.

Die Hindurchleitung von Spülfluid in Schritt b) erfolgt durch Unterdruck. Dazu wird mittels des Unterdruckaggregats ein Unterdruck im Verband erzeugt. Der Betrieb des Unterdruckaggregats erfolgt zu diesem Zweck gleichzeitig mit der Hindurchleitung des Spülfluids. Das Unterdruckaggregat wird dabei entweder stetig, also mit im Wesentlichen konstanter Leistung, oder intermittierend, also mit zeitweilig aussetzender oder nachlassender Leistung, betrieben.

In einer Ausführungsform des Verfahrens gehen Schritt a) die folgenden Schritte voran:
a1) Aufbau von Unterdruck in dem Verband durch Betrieb des Unterdruckaggregats,
a2) Befüllen der Instillationsleitung und der Hauptleitung mit Instillationsfluid.

Vorzugsweise wird zwischen den Schritten a1) und a2) die Fluidverbindung in der Abflussleitung unterbrochen. Auf diese Weise kann ein gegebenenfalls an die Abflussleitung angeschlossener Sammelbehälter belüftet werden, während im Verband weiterhin Unterdruck anliegt bzw. während der Verband mit Instillationsfluid befüllt wird.

Zusätzlich kann nach Schritt a1) eine Messung des Unterdrucks für eine vorherbestimmte Dauer erfolgen, um die Dichtheit des Verbands zu prüfen.

Der in Schritt a1) im Verband erzeugte Unterdruck dient in den darauffolgenden Schritten a2) und a) zur Erzeugung eines Fluidflusses in dem Instillationsfluid, mit dem das Instillationsfluid zunächst in die Hauptleitung und anschließend in den Verband gesaugt wird.

In einer weiteren Ausführungsform folgen auf Schritt b) die folgenden zusätzlichen Schritte:
c) Aufbau von Unterdruck in dem Verband durch Betrieb des Unterdruckaggregats,
d) Hindurchleiten eines Spülfluids durch die Spülleitung, die Hauptleitung, den Verband und die Abflussleitung.

Schritt c) stellt die Unterdruckbehandlung dar. Während dieses Schrittes wird gegebenenfalls im Verband vorhandenes Instillationsfluid abgesaugt. Vorzugsweise wird das Unterdruckaggregat während Schritt c) intermittierend betrieben, um Intervalle von höherem oder niedrigerem Unterdruck im Verband zu erzeugen. Es ist auch möglich, die Schritte c) und d) mehrmals aufeinander folgend durchzuführen. In einer weiteren Ausführungsform werden die Schritte a) bis d) mehrfach wiederholt. Zusätzlich kann bei dieser Variante bei der ersten Durchführung von Schritt a) die Füllmenge des Instillationsfluids im Verband bestimmt werden. Die Bestimmung erfolgt vorzugsweise visuell durch den Benutzer der Vorrichtung. Die somit bestimmte Füllmenge gibt das maximale Füllvolumen des Verbands an. Bei nachfolgenden Wiederholungen von Schritt a) wird der Verband jeweils mit der zuvor bestimmten Füllmenge von Instillationsfluid befüllt, ohne dass die Füllmenge erneut bestimmt werden muss.

Sofern bei dieser Ausführungsform vor Schritt a) eine Messung des Unterdrucks für eine vorherbestimmte Dauer erfolgt, um die Dichtheit des Verbands zu prüfen, reicht es aus, diese Messung nur vor der ersten Durchführung von Schritt a) auszuführen.

### BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine schematische Ansicht einer erfindungsgemäßen Vorrichtung;
- Fig. 2A-C: schematische Ansichten von alternativen Ausführungsformen der erfindungsgemäßen Vorrichtung;
- Fig. 3: schematische Ansicht einer alternativen Ausführungsformen der erfindungsgemäßen Vorrichtung und
- Fig. 4A-B: schematische Ansichten von alternativen Ausführungsformen der erfindungsgemäßen Vorrichtung.
- Fig. 5: eine schematische Ansicht einer Variante der erfindungsgemäßen Vorrichtung;
- Fig. 6: eine schematische Ansicht einer Variante der erfindungsgemäßen Vorrichtung;

### BESCHREIBUNG BEVORZUGTER AUSFÜHRUNGSFORMEN

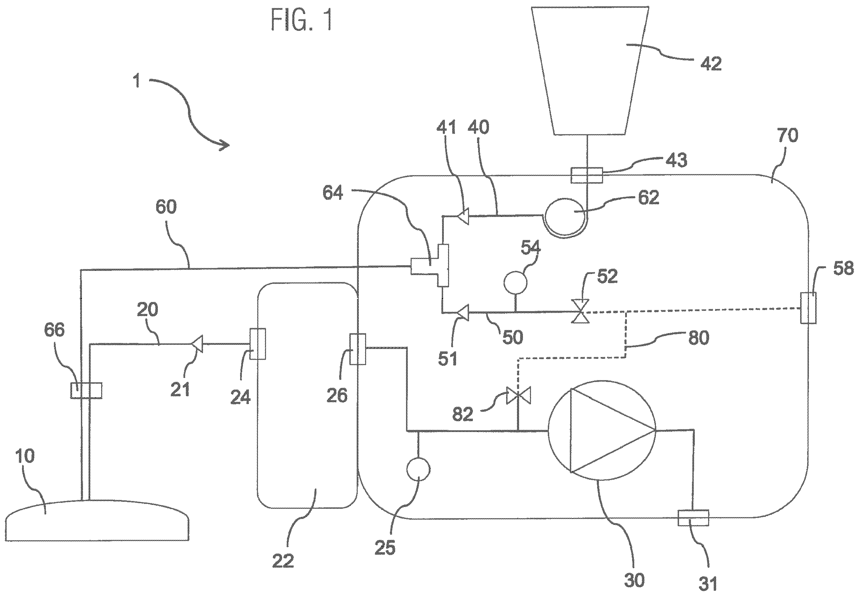

Die in Fig. 1 gezeigte Vorrichtung 1 umfasst einen Verband 10 zum Abdecken einer Wunde. Der Verband 10 umfasst typischerweise eine flexible elastische Kunststoffabdeckung, mit der eine Wunde unter Bildung eines Wundraums fluiddicht, also gas- und flüssigkeitsdicht, abgedeckt werden kann. Der Verband 10 umfasst ferner eine in der Zeichnung nicht dargestellte Wundauflage, die innerhalb des Wundraums angeordnet ist.

Der Verband 10 ist über einen in der Zeichnung nicht gezeigten Anschluss mit einer Abflussleitung 20 und einer Hauptleitung 60 jeweils fluidleitend verbunden. Der Anschluss erleichtert das Anschließen und Entfernen der Abflussleitung 20 und der Hauptleitung 60 an den bzw. von dem Verband 10.

Mit einem zweiten, dem Verband abgewandten Leitungsende mündet die Abflussleitung 20 in das Innere eines Sammelbehälters 22. Der Sammelbehälter 22 dient zur Aufnahme des von der Wunde abgesaugten Fluids. Die Absaugung des Fluids erfolgt durch Unterdruck, der von einem mit dem Sammelbehälter fluidleitend verbundenen Unterdruckaggregat 30 erzeugt wird. Das Unterdruckaggregat ist innerhalb eines Gehäuses 70 angeordnet.

Der Sammelbehälter 22 ist in der in Fig. 1 gezeigten Ausführungsform an dem Gehäuse 70 angebracht. Er kann als Einwegartikel ausgeführt und bei Erreichen des Maximalfüllstands von dem Gehäuse abgenommen und mitsamt des gesammelten Fluids entsorgt werden. Der Sammelbehälter kann allerdings auch wiederverwendbar sein, indem er abgenommen, gereinigt und erneut an dem Gehäuse angebracht werden kann. Vorzugsweise ist der Sammelbehälter 22 über eine Schnappverbindung am Gehäuse 70 befestigt.

Die Abflussleitung 20 ist über einen lösbaren Anschluss 24 mit dem Sammelbehälter 22 verbunden. Auf der dem Sammelbehälter 22 abgewandten Seite des Anschlusses ist ein Abflussventil 21 vorgesehen, welches den Fluidfluss in Richtung des Verbandes blockiert, jedoch Fluidfluss in Richtung des Sammelbehälters zulässt.

Die Vorrichtung umfasst außerdem ein Gehäuse 70, das das Unterdruckaggregat 30 in sich aufnimmt. Das Unterdruckaggregat 30 steht mit dem Sammelbehälter 22 in Fluidkommunikation und kann somit im Sammelbehälter 22 einen Unterdruck erzeugen, der sich über die Abflussleitung 20 bis zum Wundraum fortsetzt. Zwischen Sammelbehälter 22 und Unterdruckaggregat 30 ist ein Filter 26 angeordnet, der eine mikrobielle Kontamination von Sammelbehälter 22 bzw. Unterdruckaggregat 30 verhindert. Zwischen Filter 26 und Unterdruckaggregat 30 ist zusätzlich ein Drucksensor 25 angeordnet.

Das Unterdruckaggregat 30 steht ferner in Fluidkommunikation mit einer Auslassöffnung 31 auf der Außenseite des Gehäuses 70.

Die Hauptleitung 60 ist mit einem Anschlusselement 64 verbunden, welches vorzugsweise innerhalb des Gehäuses angeordnet ist. Das Anschlusselement 64 ist beispielsweise in T- oder Y-förmiges Verbindungsglied mit insgesamt drei Anschlüssen für die Hauptleitung 60, die Instillationsleitung 40 und die Spülleitung 50. Das Anschlusselement 64 kann so angeordnet sein, dass der Anschluss für die Hauptleitung 60 an der Außenseite des Gehäuses 70 freiliegt und von außen zugänglich ist. In diesem Fall kann die Hauptleitung lösbar mit dem Anschlusselement 64 verbunden sein, so dass die Hauptleitung 60 leicht ausgetauscht werden kann.

Die Abflussleitung 20 und die Hauptleitung 60 verlaufen beide durch eine Kopplung 66. Zwischen der Kopplung 66 und dem Verband 10 bilden die Abflussleitung 20 Hauptleitung 60 gemeinsam eine zweilumige Schlauchverbindung. Die Kopplung 66 erlaubt ein einfaches Anschließen und Entfernen der zweilumigen Schlauchverbindung an die bzw. von der Vorrichtung 1. Die zweilumige Schlauchverbindung bildet eine Einheit mit dem Verband 10. Diese Einheit kann als Einwegartikel gestaltet sein, der nach Gebrauch entsorgt werden kann.

Das Anschlusselement 64 ist mit dem zweiten seiner Anschlüsse mit der Instillationsleitung 40 verbunden. In der Instillationsleitung 40 kann ein Ventil 41 angeordnet sein, um einen Rückfluss von Instillations- oder Spülfluid in die Instillationsleitung 40 zu verhindern. Das Ventil kann zusätzlich die Funktion haben, ein ungewolltes Durchtreten des Instillationsfluids in Richtung des Verbands 10 zu verhindern. Die Instillationsleitung 40 ist mit einem Instillationsbehälter 42 verbunden, denn der in der Zeichnung gezeigten Vorrichtung außerhalb des Gehäuses 70 angeordnet ist. An der Außenseite des Gehäuses 70 ist ein Anschluss 43 angeordnet, mit dem die Instillationsleitung 40 und der Instillationsbehälter 42 miteinander verbunden werden können. Vorzugsweise ist der Anschluss 43 lösbar, sodass der Instillationsbehälter 42 leicht ausgetauscht werden kann.

In der in der Zeichnung gezeigten Vorrichtung handelt es sich bei dem Instillationsbehälter 42 um einen Kunststoffbeutel oder eine kopfüber angeordnete Kunststoffflasche, die oberhalb des Gehäuses 70 und oberhalb des Verbands 10 angebracht ist. Auf diese Weise wird ein Fluidfluss durch hydrostatischen Druck erzeugt.

Es ist auch möglich, dass der Instillationsbehälter 42, ähnlich wie der Sammelbehälter 22, ankoppelbar am Gehäuse 70 angebracht ist. In diesem Fall entfällt die Schlauchverbindung zwischen dem Anschluss 43 und dem Instillationsbehälter 42.

An der Instillationsleitung 40 ist eine Peristaltikpumpe 62 angeordnet, die einen Fluidfluss in dem Instillationsfluid erzeugt. Die Peristaltikpumpe 62 ist steuerbar, sodass der Fluidfluss im Verlauf der Behandlung variiert werden kann. Peristaltikpumpe 62 ist auf der dem Anschlusselement 64 abgewandten Seite des Ventils 41 angeordnet.

In der in der Zeichnung gezeigten Ausführungsform ist die Peristaltikpumpe 62 innerhalb des Gehäuses 70 angeordnet. Es ist allerdings ebenso möglich, die Peristaltikpumpe 62 an der Außenseite des Gehäuses 70, oder im bzw. am Sammelbehälter 22 anzubringen, oder als separates Modul auszuführen.

Das Anschlusselement 64 ist mit dem dritten seiner Anschlüsse mit der Spülleitung 50 verbunden. In der Spülleitung 50 kann ein Ventil 51 angeordnet sein, dass ein Rückfluss von Instillations- oder Spülfluid in die Spülleitung 50 verhindert. Vorzugsweise handelt es sich bei dem Ventil 51 um eine Membran, die bei Kontakt mit Feuchtigkeit abdichtet. Auf der dem Anschlusselement 64 abgewandten Seite des Ventils 51 ist ein Drucksensor 54 angeordnet, mit dem der Unterdruck in der Spülleitung 50, der daran angeschlossenen Hauptleitung 60 und dem Wundraum gemessen werden kann. Auf der dem Anschlusselement 64 abgewandten Seite des Drucksensor 54 ist ein Belüftungsventil 52 angeordnet, dass die Fluidkommunikation durch die Spülleitung 50 öffnen oder unterbrechen kann. Die Spülleitung 50 steht über einen Anschluss 58 in Fluidkommunikation mit der Umgebungsluft. Auf diese Weise ist ein belüfteten der Spülleitung 50 und der daran angeschlossenen Hauptleitung 60 möglich. Der Anschluss 58 umfasst ein Filterelement, um eine Kontamination der Spülleitung 50 mit Mikroorganismen aus der Umgebungsluft zu verhindern.

Die Spülleitung 50 steht über eine Bypassleitung 80 in Fluidkommunikation mit dem Unterdruckaggregat 30 und dem daran angeschlossenen Fluidsammelbehälter 22. Die Bypassleitung 80 kann über ein Bypassventil 82 geöffnet oder geschlossen werden. Auf diese Weise ist auch ein direktes Belüfteten des Unterdruckaggregats 30 und des Fluid Sammelbehälter 22 möglich. Das Ventil 21 verhindert dabei gleichzeitiges Belüfteten der Abflussleitung 20 und des Wundraums. Somit können das Unterdruckaggregat 30 und der Sammelbehälter 22 bei gleichzeitig im Wundraum anliegenden Unterdruck belüftet werden.

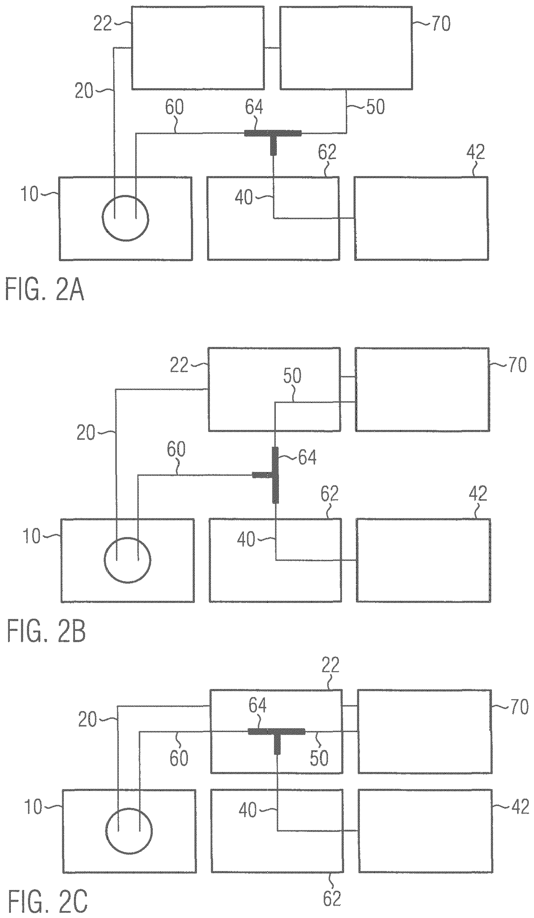

Die Figuren 2A bis 2C zeigen unterschiedliche Varianten der erfindungsgemäßen Vorrichtung in schematischer Darstellung. Die Ausführungsformen gemäß dieser Varianten umfassen wie die oben beschriebene Ausführungsform ein Gehäuse 70, das ein in den Zeichnungen nicht gezeigtes Unterdruckaggregat in sich aufnimmt. Das Unterdruckaggregat steht mit einem Sammelbehälter 22 in Fluidkommunikation. Der Sammelbehälter 22 ist über die Abflussleitung 20 an den Verband 10 angeschlossen. Der Verband 10 ist über die Hauptleitung 60 und ein Anschlusselement 64 an eine Instillationsleitung 40 und eine Spülleitung 50 angeschlossen. Die Spülleitung 50 mündet jeweils in das Gehäuse 70.

Der Verband 10 weist in diesen Varianten jeweils einen einzelnen Anschluss auf, an den sowohl Abflussleitung 20 als auch Hauptleitung 60 angeschlossen sind.

Anders als in der zuvor gezeigten Ausführungsform ist die Peristaltikpumpe 62 in diesem Varianten jeweils außerhalb des Gehäuses 70 angeordnet und bildet ein separates Modul der Vorrichtung. Dieses Modul kann beispielsweise wie in US 2019/0275219 A1 ausgeführt sein.

Die Varianten unterscheiden sich jeweils durch die Anordnung des Anschlusselements 64. Gemäß den Figuren 2A und 2B ist das Anschlusselement 64 zwischen dem Gehäuse 70, dem Sammelbehälter 22 und der Peristaltikpumpe 62 angeordnet. Gemäß Fig. 2A verlaufen sowohl Instillationsleitung 40 als auch Spülleitung 50 außerhalb des Sammelbehälters 22. Gemäß Fig. 2B verläuft die Spülleitung 50 durch den Sammelbehälter 22. In der Variante gemäß Fig. 2C ist das Anschlusselement 64 innerhalb des Sammelbehälters 22 angeordnet, so dass Spülleitung 50 und Instillationsleitung zumindest abschnittsweise innerhalb des Sammelbehälters 22 verlaufen.

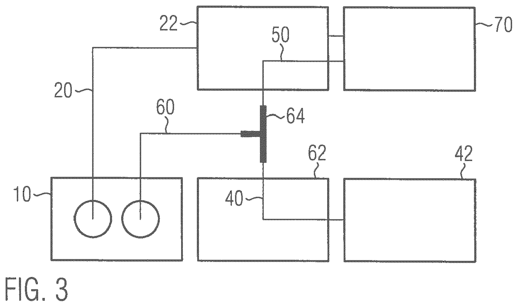

Die Figur 3 zeigt eine weitere Variante der erfindungsgemäßen Vorrichtung in schematischer Darstellung. Diese Variante entspricht der Ausführungsform nach Fig. 2B, allerdings weist der Verband 10 hier zwei getrennte Anschlüsse für die Abflussleitung 20 bzw. die Hauptleitung 60 auf.

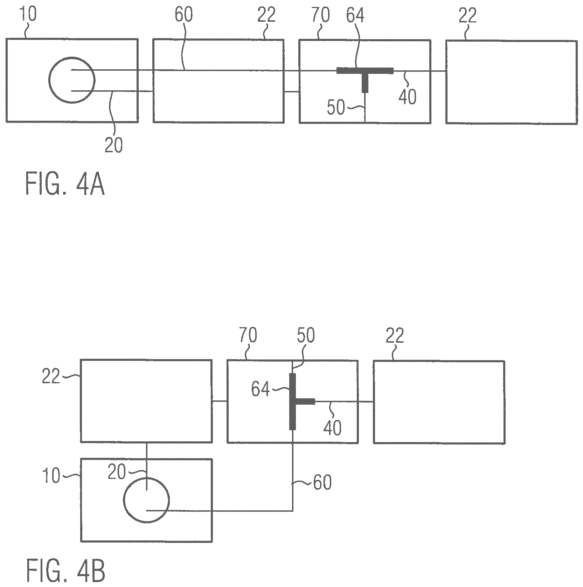

Die Figuren 4A und 4B zeigen weitere Varianten der erfindungsgemäßen Vorrichtung in schematischer Darstellung. Bei diesen Varianten nimmt das Gehäuse 70 sowohl die in den Zeichnungen nicht gezeigte Peristaltikpumpe als auch das Unterdruckaggregat in sich auf. Zudem ist das Anschlusselement 64 im Unterschied zu den oben gezeigten Varianten innerhalb des Gehäuses 70 angeordnet. Die beiden Varianten entsprechen insofern der Ausführungsform gemäß Fig. 1. Der Unterschied zwischen beiden Varianten ergibt sich dadurch, dass die Hauptleitung gemäß Fig. 4A durch den Sammelbehälter 22 hindurch verläuft, während sie gemäß Fig. 4B außerhalb des Sammelbehälters 22 verläuft.

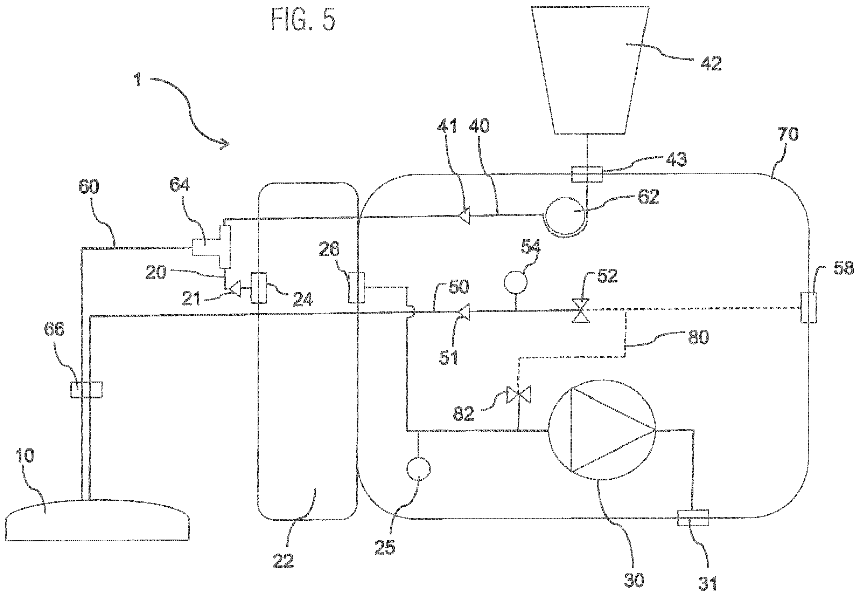

Figur 5 zeigt eine weitere Variante der erfindungsgemäßen Vorrichtung. Diese Variante entspricht bis auf die folgenden Unterschiede der in Figur 1 gezeigten Ausführungsform. Anders als bei der in Figur 1 gezeigten Ausführungsform ist das Anschlusselement 64 in Figur 5 mit der Instillationsleitung 40 und der Abflussleitung 20 verbunden. Dadurch werden die Instillationsleitung 40 und die Abflussleitung 20 gemeinsam mit der einlumigen Hauptleitung 60 verbunden. Durch das Lumen der Hauptleitung 60 werden auf diese Weise sowohl das Instillationsfluid der Wunde zugeleitet als auch Wundsekret von der Wunde abgesaugt. Das Anschlusselement weist vorzugsweise eine Vorrichtung auf, mit der wechselweise eine Fluidverbindung zur Abflussleitung 20 oder zur Instillationsleitung 40 hergestellt und die Fluidverbindung zur jeweils anderen Leitung unterbrochen werden kann.

Die Spülleitung 50 bildet in dieser Ausführungsform eine separate Leitung, die getrennt von der Abflussleitung 20 und der Instillationsleitung 40 zum Verband 10 geführt wird.

Die Druckmessung erfolgt bei dieser Ausführungsform durch den in der Spülleitung 50 angeordneten Drucksensor 54 und/oder den in der Fluidverbindung zwischen Sammelbehälter 22 und Unterdruckaggregat 30 angeordneten Drucksensor 25.

In einem Abschnitt bilden die Spülleitung 50 und die Hauptleitung 60 eine zweilumige Schlauchverbindung. Dieser Abschnitt verläuft zwischen der Kopplung 66 und dem Verband 10.

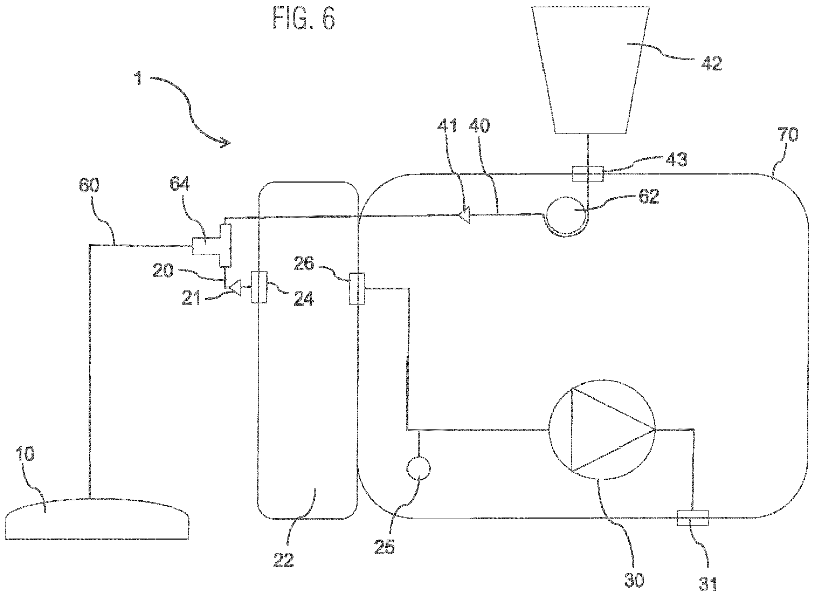

Figur 6 zeigt eine weitere Variante der erfindungsgemäßen Vorrichtung. Diese Variante gleicht der Variante gemäß Figur 5 bis auf die Tatsache, dass die Vorrichtung gemäß Figur 6 keine Spülleitung aufweist. Ebenso wie in Figur 5 umfasst die Vorrichtung jedoch eine Abflussleitung 20 und eine Instillationsleitung 40, die beide mit dem Anschlusselement 64 und über dieses mit der Hauptleitung 60 verbunden sind. Durch das Lumen der Hauptleitung 60 werden auf diese Weise sowohl das Instillationsfluid der Wunde zugeleitet als auch Wundsekret von der Wunde abgesaugt. Der Verband 10 ist in dieser Ausführungsform nur mit einer einzelnen Leitung, der Hauptleitung 60 verbunden.

Die Druckmessung erfolgt bei dieser Ausführungsform durch den in der Fluidverbindung zwischen Sammelbehälter 22 und Unterdruckaggregat 30 angeordneten Drucksensor 25.

### BEZUGSZEICHENLISTE

- 1: Vorrichtung
- 10: Verband
- 20: Abflussleitung
- 21: Abflussventil
- 22: Sammelbehälter
- 24: Anschluss
- 25: Drucksensor
- 26: Filter
- 30: Unterdruckaggregat
- 31: Auslassöffnung
- 40: Instillationsleitung
- 41: Ventil
- 42: Instillationsbehälter
- 43: Anschluss
- 50: Spülleitung
- 51: Ventil
- 52: Belüftungsventil
- 54: Drucksensor
- 56: Belüftungsvorrichtung
- 58: Filter
- 60: Hauptleitung
- 62: Pumpe
- 64: Anschlusselement
- 66: Kopplung
- 70: Gehäuse
- 80: Bypassleitung
- 82: Bypassventil

## Patentansprüche

1. Vorrichtung (1) zur Unterdruckbehandlung und Instillation von Wunden umfassend
einen Verband (10) zur fluiddichten Abdeckung einer Wunde,
eine Abflussleitung (20) zum Absaugen von Fluid von der Wunde,
ein Unterdruckaggregat (30) zum Erzeugen eines Unterdrucks in der Abflussleitung,
eine Instillationsleitung (40) zum Bereitstellen eines Instillationsfluids,
eine optionale Spülleitung (50) zum Bereitstellen eines Spülfluids,
eine mit dem Verband (10) verbundene Hauptleitung (60),
**dadurch gekennzeichnet, dass**
die Hauptleitung (60) ein einzelnes Lumen aufweist und über ein Anschlusselement (64) mit zwei der folgenden Leitungen, Abflussleitung (20), Instillationsleitung (40) und Spülleitung (50), verbindbar ist.

2. Vorrichtung nach Anspruch 1, wobei
die Hauptleitung (60) über das Anschlusselement (64) entweder mit der Abflussleitung (20) und der Instillationsleitung (40) oder mit der Instillationsleitung (40) und der Spülleitung (50) verbindbar ist.

3. Vorrichtung nach Anspruch 2, wobei
die Vorrichtung (1) die Spülleitung (50) aufweist und
die Hauptleitung (60) über das Anschlusselement (64) mit der Instillationsleitung (40) und der Spülleitung (50) verbindbar ist.

4. Vorrichtung nach einem der vorhergehenden Ansprüche, weiterhin umfassend
einen Drucksensor (25, 54) zur Messung des Drucks in der Abflussleitung (20), der Instillationsleitung (40), der Spülleitung (50) und/oder der Hauptleitung (60).

5. Vorrichtung nach einem der vorhergehenden Ansprüche, weiterhin umfassend
ein in der Instillationsleitung (40) angeordnetes Ventil (41), das einen Rückfluss von Fluid in die Instillationsleitung (40) verhindert.

6. Vorrichtung nach einem der vorhergehenden Ansprüche, weiterhin umfassend
einen mit der Instillationsleitung (40) verbundenen Instillationsbehälter (42) zur Aufnahme eines Instillationsfluids.

7. Vorrichtung nach einem der vorhergehenden Ansprüche, weiterhin umfassend
ein in der Spülleitung (50) angeordnetes Ventil (51), das einen Rückfluss von Fluid in die Spülleitung (50) verhindert.

8. Vorrichtung nach einem der vorhergehenden Ansprüche, weiterhin umfassend
ein in der Spülleitung (50) angeordnetes Belüftungsventil (52), mit dem die Fluidkommunikation innerhalb der Spülleitung (50) unterbrochen werden kann.

9. Vorrichtung nach einem der vorhergehenden Ansprüche, weiterhin umfassend
eine in der Instillationsleitung (40), der Spülleitung (50) oder der Hauptleitung (60) angeordnete Pumpe (62) zur Erzeugung eines Fluidflusses.

10. Vorrichtung nach einem der vorhergehenden Ansprüche, weiterhin umfassend
eine Einheit zur Steuerung des Fluidflusses durch die Hauptleitung (60), so dass wechselweise entweder das Instillationsfluid oder das Spülfluid durch die Hauptleitung (60) geleitet werden.

11. Vorrichtung nach einem der vorhergehenden Ansprüche, weiterhin umfassend
einen mit der Abflussleitung (20) verbundenen Sammelbehälter (22) zur Aufnahme des von der Wunde abgesaugten Fluids.

12. Vorrichtung nach einem der vorhergehenden Ansprüche, weiterhin umfassend
eine Bypassleitung (80), die eine Fluidkommunikation zwischen der Spülleitung (50) und dem Unterdruckaggregat (30) herstellt.

13. Verfahren zum Betrieb einer Vorrichtung (1) zur Unterdruckbehandlung und Instillation von Wunden gemäß einem der Ansprüche 3 bis 11, umfassend die folgenden Schritte:
a) Befüllen des Verbands mit Instillationsfluid aus der Hauptleitung (60),
b) Hindurchleiten eines Spülfluids durch die Spülleitung (50), die Hauptleitung (60), den Verband (10) und die Abflussleitung (20) bei gleichzeitigem stetigen oder intermittierenden Betrieb des Unterdruckaggregates.

14. Verfahren gemäß Anspruch 13, wobei Schritt a) die folgenden Schritte vorangehen:
a1) Aufbau von Unterdruck in dem Verband (10) durch Betrieb des Unterdruckaggregats (30), und
a2) Befüllen der Instillationsleitung (40) und der Hauptleitung (60) mit Instillationsfluid.

15. Verfahren gemäß einem der Ansprüche 13 und 14, wobei Schritt b) die folgenden Schritte folgen:
c) Aufbau von Unterdruck in dem Verband (10) durch Betrieb des Unterdruckaggregats (30),
d) Hindurchleiten eines Spülfluids durch die Spülleitung (50), die Hauptleitung (60), den Verband (10) und die Abflussleitung (20).

16. Verfahren gemäß Anspruch 15, wobei
die Schritte a) bis d) mehrfach wiederholt werden,
bei der ersten Durchführung von Schritt a) die Füllmenge des Instillationsfluids im Verband (10) bestimmt wird und
bei den weiteren Wiederholungen von Schritt a) der Verband (10) mit der vorherbestimmten Füllmenge von Instillationsfluid befüllt wird.
